# EUROPEAN PATENT APPLICATION

(11) **EP 1 785 411 A1**
(43) Date of publication of application: **16.05.2007**
(21) Application number: 06022313.8
(22) Date of filing: 25.10.2006
(51) Int. Cl.: C07C 209/00, C07C 209/84, C07C 211/63

(54) **Protriptyline hydrochloride crystalline form**

(30) Priority: 14.11.2005 IT MI20052170
(71) Applicant: Dipharma S.p.A., 33036 Mereto di Tomba (Udine) (IT)
(72) Inventor: Ventimiglia, Gianpiero, 20092 Cinisello Balsamo(MI) (IT); Magrone, Domenico, 20128 Milano (IT); Allegrini, Pietro, 20097 San Donato Milanese (MI) (IT); Razzetti, Gabriele, 20099 Sesto S. Giovanni (MI) (IT)
(74) Representative: Minoja, Fabrizio

(57) **Abstract**

Protriptyline hydrochloride crystalline Form B, a process for its preparation, a pharmaceutical composition containing it the use thereof.

## Description

### FIELD OF THE INVENTION

The present invention relates to a novel crystalline form of protriptyline hydrochloride, a process for its preparation, pharmaceutical compositions containing it and its use.

### TECHNOLOGICAL BACKGROUND

Protriptyline, N-methyl-5H-dibenzo[a,d]-cycloheptene-5-propanamine, of formula: 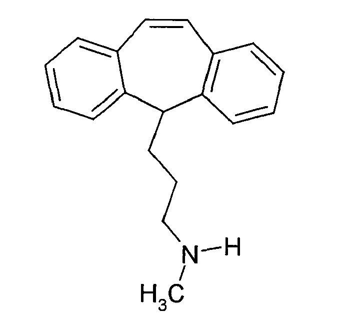 is known from GB 1 008 263, and its hydrochloride is used in therapy as an antidepressant agent.

Different crystalline forms of biologically active compounds, in particular polymorphic forms, can be useful in pharmaceutical technique and therapy, thanks to different physical properties, such as flowability of the ground powdered product. Powder flowability is in fact an important factor for the preparation of solid pharmaceutical forms, such as tablets or capsules. Dissolution time of the medicament in aqueous fluids - such as stomach fluid - is also an important therapeutical factor, as it affects bioavailability of the drug and therefore its blood concentration. This can be advantageous for patients, as it allows, for example, to reduce dosages and to prolong intervals between administrations.

There is therefore the need for polymorphic forms of biologically active compounds.

### SUMMARY OF THE INVENTION

It has now been found that protriptyline hydrochloride can exist not only in the known form commercially available at present, herein referred to as Form A, but also in another crystalline form, stable at room temperature, herein referred to as Form B.

Therefore, the present invention relates to said Form B of protriptyline hydrochloride, a process for its preparation, pharmaceutical compositions containing it and its use.

### BRIEF DISCLOSURE OF THE FIGURES

Crystalline forms A and B were characterized with the known XRPD technique (X-ray powder diffraction). X-ray diffraction spectra (XRPD) were recorded with an APD 2000 θ/θ automatic diffractometer for powders and liquids (Ital-Structures), under the following operative conditions: CUKα radiation (X = 1.5418 Å), scanning with angular interval 3-40°, with angular step of 0.03° for 1 sec.
Figure 1. XRPD spectrum of protriptyline hydrochloride Form A, wherein the most intense diffraction peaks fall at 9.08; 11.78; 16.46; 19.31; 23.69; 25.10 ± 0.2 in 2θ.
Figure 2. XRPD spectrum of protriptyline hydrochloride Form B.

### DETAILED DISCLOSURE OF THE INVENTION

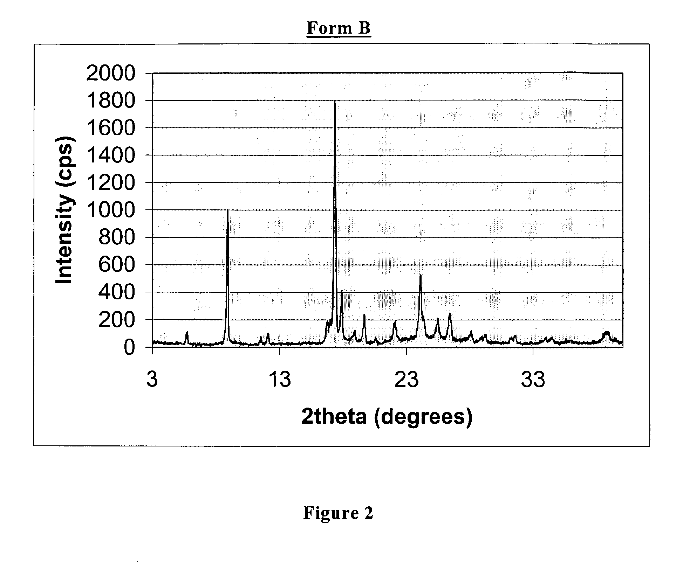

As a first object, the invention provides protriptyline hydrochloride in the crystalline form, in the following defined Form B, having the XRPD spectrum substantially as reported in Figure 2, wherein the most intense diffraction peaks fall at 8.87; 17.36; 17.87; 24.08 ± 0.2 in 2θ.

Said Form B can be prepared by a process which comprises:
- suspending protriptyline free base in a keto solvent;
- adding hydrochloric acid gas to the resulting suspension; and
- recovering the resulting solid.

Examples of keto solvents are compounds of formula R₁(CO)R₂, wherein each of R₁ and R₂ is, independently, a straight or branched C₁-C₄ alkyl group, preferably acetone, methyl ethyl ketone, diethyl ketone and methyl isobutyl ketone, more particularly acetone. The concentration of protriptyline free base in the starting suspension can approx. range from 5 to 15%, preferably from 8 to 12%. The resulting suspension is added with hydrochloric acid gas, in sufficient amount to saturate the solvent, at a temperature approx. ranging preferably from 20 to 25°C. Protriptyline hydrochloride Form B is separated from the resulting suspension, and it can be recovered with a conventional technique, such as filtration or centrifugation, followed by washing with the keto solvent used above and finally drying under vacuum, at a temperature depending on the used solvent, as it is well-known.

Protriptyline hydrochloride Form B can thus be obtained with purity at least equal to or higher than 99.9%.

The terms "about" and "approximately", as used herein, mean 10% more or less.

A further object of the invention is a process for the preparation of protriptyline hydrochloride Form A comprising:
- dissolving protriptyline hydrochloride Form B in an alcoholic solvent;
- cooling said solution to crystallize protriptyline hydrochloride Form A;
- recovering the resulting solid.

Protriptyline hydrochloride Form B is dissolved in the hot, typically at a temperature around the reflux temperature of the alcohol used. The alcoholic solvent can be for example a straight or branched C₁-C₄ alkanol, tipically methanol, ethanol, isopropanol or 1-butanol, preferably isopropanol. The concentration of protriptyline hydrochloride Form B in the starting suspension can approx. range from 4 to 8%, preferably approx. from 5 to 6%. The resulting solution is cooled at a temperature preferably ranging from 15 to 20°C, thereby crystallizing protriptyline hydrochloride Form A, that can be recovered according to one of the above mentioned conventional techniques.

An object of the invention is also a purification process for the preparation of protriptyline hydrochloride Form A with purity at least equal to or higher than 99.9%, comprising the preparation of protriptyline hydrochloride Form B from protriptyline free base and its conversion to protriptyline hydrochloride Form A. The preparation of protriptyline hydrochloride Form B and its conversion to protriptyline Form A can be carried out as reported above.

The invention therefore provides protriptyline hydrochloride Form A with purity at least equal to or higher than 99.9%, as obtainable by the process reported above.

If desired, the resulting protriptyline hydrochloride Form A can be converted to protriptyline free base, which can in turn be transformed into another pharmaceutically acceptable salt, in particular protriptyline hydrochloride Form B. The conversion of protriptyline Form A to protriptyline free base or in a pharmaceutically acceptable salt, in particular protriptyline hydrochloride Form B, can be carried out according to known methods or as herein reported.

Protriptyline free base or a pharmaceutically acceptable salt thereof, in particular protriptyline hydrochloride Form B, can therefore be obtained in good yields and high purity, at least equal to or higher than 99.9%, which meets the regulatory requirements for the preparation of galenic formulations.

The invention also relates to a process for the preparation of protriptyline hydrochloride Form A, which process comprises:
- suspending protriptyline free base in a keto solvent;
- adding a hydrochloric acid aqueous solution to the resulting suspension;
- cooling the suspension to crystallize protriptyline hydrochloride Form A; and
- recovering the resulting solid.

A keto solvent can be for example a compound of formula R₁(CO)R₂, wherein R₁ and R₂ are as defined above. Examples of keto solvents are acetone, methyl ethyl ketone, diethyl ketone and methyl isobutyl ketone, preferably acetone. The concentration of protriptyline free base in the starting suspension can approx. range from 5 and to 15%, preferably from 8 to 12%. Hydrochloric acid is added to the resulting suspension at a temperature preferably comprised between from 20 and 25°C. The concentration of the hydrochloric acid aqueous solution typically ranges from 5 to 37%, and is preferably 37%. The ratio of added hydrochloric acid to starting base approx. ranges from 1 to 1.5 equivalents, more preferably approx. from 1 to 1.2 equivalents. The resulting suspension, after optional seeding with a crystalline seed, is cooled to a temperature preferably ranging approx. from 15 to 20°C, thereby precipitating protriptyline hydrochloride Form A. The latter can be recovered with known techniques, as reported above.

Protriptyline hydrochloride Form B can be used for the treatment of those pathologies in which protriptyline hydrochloride Form A is used.

An object of the invention is also a pharmaceutical composition comprising protriptyline hydrochloride Form B as the active ingredient, and an excipient and /or carrier thereof.

Said pharmaceutical composition can further comprise as active ingredient, protriptyline hydrochloride Form A in amounts approx. ranging from 0.1 to 99.9%, preferably from 10 to 90%.

A pharmaceutical composition of the invention can be formulated with known methods in any pharmaceutical form known for the administration to mammals, including humans.

The following examples illustrate the invention.

### EXAMPLE 1

### Preparation of protriptyline hydrochloride Form A

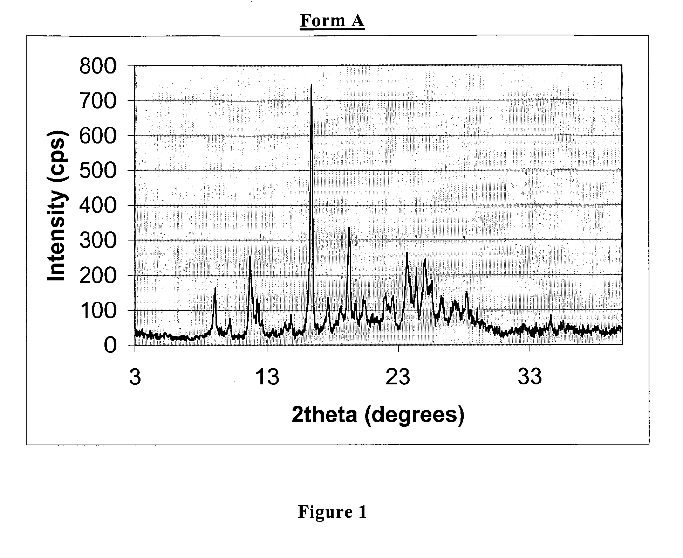

A 2000 ml round-bottom flask, equipped with mechanical stirrer, thermometer and dropping funnel, is loaded with 164 of protriptyline free base and 1500 ml of acetone. 70 g of 37% HCl are dropwise added and the mixture is cooled to 5°C to crystallize the product, which is recovered by filtration, washed with acetone and dried under vacuum at a temperature of 50°C, thereby obtaining 120 g of protriptyline hydrochloride Form A (yield: 64%) having the XRPD spectrum substantially as reported in Figure 1.

### EXAMPLE 2

### Preparation of protriptyline hydrochloride Form B

A 250 ml round-bottom flask, equipped with magnetic stirrer, thermometer and gas inlet tube for HCl, is loaded with 12 g of protriptyline free base and 100 ml of acetone. HCl gas is bubbled through the resulting solution, keeping a temperature of 20-25°C, until saturation. The precipitated white solid is filtered off, washed with acetone and dried under vacuum at room temperature, thereby obtaining 13 g of protriptyline hydrochloride Form B (yield: 95%), having the XRPD spectrum substantially as reported in Figure 2.

### EXAMPLE 3

### Preparation of protriptyline hydrochloride Form A

A 250 ml round-bottom flask, equipped with magnetic stirrer and thermometer, is loaded with 10 g of protriptyline hydrochloride Form B and 100 ml of isopropanol. The mixture is refluxed (82°C) until complete dissolution, then cooled to room temperature (20-25°C). The crystallized white solid is filtered off, washed with isopropanol and dried under vacuum at a temperature of 60°C, thereby obtaining 8.5 g of protriptyline hydrochloride Form A (yield: 85%; purity 99.95%), having the XRPD spectrum substantially as reported in Figure 1.

## Claims

1. Protriptyline hydrochloride crystalline Form B, having an XRPD spectrum with the most intense diffraction peaks at 8.87; 17.36; 17.87; 24.08 ± 0.2 in 2θ*.*

2. A process for the preparation of protriptyline hydrochloride Form B, as defined in claim 1, comprising:
• suspending protriptyline free base in a keto solvent;
• adding hydrochloric acid gas to the resulting suspension; and
• recovering the resulting solid.

3. A process according to claim 2, wherein the keto solvent is a compound of formula R₁(CO)R₂, wherein each of R₁ and R₂ is, independently, a straight or branched C₁-C₄ alkyl group.

4. A process according to claim 2, wherein the concentration of protriptyline free base in the starting suspension approx. ranges from 5 to 15%.

5. Protriptyline hydrochloride Form B, with purity equal to or higher than 99.9%, as obtainable according to the process of claim 2.

6. A process for the preparation of protriptyline hydrochloride Form A, comprising:
• dissolving protriptyline hydrochloride Form B in an alcoholic solvent;
• cooling said solution to crystallize protriptyline hydrochloride Form A;
• recovering the resulting solid.

7. A purification process for the preparation of protriptyline hydrochloride Form A with purity equal to or higher than 99.9%, comprising the preparation of protriptyline hydrochloride Form B from protriptyline free base, according to the process of claim 2, and its conversion to protriptyline hydrochloride Form A, according to the process of claim 6.

8. Protriptyline hydrochloride Form A with purity at least equal to or higher than 99.9%.

9. Protriptyline free base, or a pharmaceutically acceptable salt thereof, with purity equal to or higher than 99.9%.

10. A process for the preparation of protriptyline hydrochloride Form A comprising:
• suspending protriptyline free base in a keto solvent;
• adding a hydrochloric acid aqueous solution to the resulting suspension;
• cooling the suspension to crystallize protriptyline hydrochloride Form A; and
• recovering the resulting solid.
